# EUROPÄISCHE PATENTSCHRIFT

(11) **EP 0 976 370 B1**
(45) Veröffentlichungstag und Bekanntmachung des Hinweises auf die Patenterteilung: **10.05.2006**
(21) Anmeldenummer: 99114636.6
(22) Anmeldetag: 26.07.1999
(51) Int. Cl.: A61F 2/60

(54) **Beinprothese**
Leg prosthesis
Prothèse de jambe

(30) Priorität: 27.07.1998 DE 29813362 U
(43) Veröffentlichungstag der Anmeldung: 02.02.2000
(73) Patentinhaber: Medi Bayreuth Weihermüller & Voigtmann GmbH & Co. KG, 95448 Bayreuth (DE)
(72) Erfinder: Shen, Hsin Sun, Keelung (TW)
(74) Vertreter: Schuhmann, Albrecht

(56) Entgegenhaltungen:
- WO-A-95/25488
- DE-C- 818 232
- DE-C- 924 473
- GB-A- 2 262 306
- US-A- 5 314 499
- US-A- 5 509 936

## Beschreibung

Die Erfindung betrifft Beinprothesen, und insbesondere ein Zwischenbaugruppe für eine Beinprothese, die im gebogenen Zustand Energie speichert und beim Entspannen Energie freisetzt.

Eine herkömmliche Einzelrohr-Zwischenbaugruppe für eine Beinprothese wird einfach wie eine Krücke zum Abstützen des Körpers des Trägers verwendet. Entwickelt wurde auch eine plattenförmige Zwischenbaugruppe, die beim Beugen der Beinprothese Energie speichert und beim Heben der Beinprothese Energie freisetzt. Diese plattenförmige Zwischenbaugruppe erleichtert dem Träger das Gehen, sie weist jedoch Nachteile auf. Bei der Montage müssen Nietlöcher gebohrt werden, so dass Teile der Zwischenbaugruppe mit Hilfe von Nieten aneinander befestigt werden können. Durch das Erzeugen von Nietlöchern in den Teilen der Zwischenbaugruppe verringert sich jedoch die Festigkeit der Zwischenbaugruppe. Sollte die Beinprothese nach dem Einsetzen nicht genau die richtige Länge haben, ist außerdem keine Korrektur auf die gewünschte Länge möglich. In der US 5314499A ist eine Lösung mit zwei Stäben als Zwischenbaugruppe beschrieben, die ebenfalls das Gehen für den Träger erleichtern soll, aber auch die genannten Nachteile hinsichtlich der Montage und des breiten Einsatzes aufweist.

Durch die Erfindung wird eine Beinprothese mit einer Zwischenbaugruppe geschaffen, die beim Beugen der Beinprothese auf wirksame Weise Energie speichert und die gespeicherte Energie beim Anheben der Beinprothese schnell wieder freisetzt. Durch die Erfindung wird ferner eine Zwischenbaugruppe für eine Beinprothese geschaffen, die abnehmbar und nachträglich längenverstellbar ist. Gemäß der Erfindung weist die Zwischenbaugruppe zwei elastische, parallel zueinander angeordnete Stäbe und ein an einem Ende an den Stäben befestigtes Anschlussstück auf, das die Stäbe mit einem Teil der Beinprothese verbindet. Das Anschlussstück weist parallele Aufnahmeöffnungen zur Aufnahme des jeweiligen Stabs, einen zwischen den Aufnahmeöffnungen ausgebildeten und diese verbindenden Spalt, eine Mehrzahl von entlang des Spalts jeweils in unterschiedlicher Höhe angeordneten Befestigungsbohrungen, und eine Mehrzahl von jeweils in den Befestigungsbohrungen befestigten Schraubenbolzen zum Schließen des Spalts und zum Festlegen der Stäbe in ihrer Lage auf.

Die Erfindung wir anhand bevorzugter Ausführungsformen unter Bezugnahme auf die Zeichnung beschrieben. In der Zeichnung zeigen
Fig. 1 eine Zwischenbaugruppe für eine Beinprothese entsprechend einer Ausführungsform der Erfindung in Explosivdarstellung,
Fig. 2 die Baugruppe nach Fig. 1 im zusammengebauten Zustand,
Fig. 3 die Baugruppe nach Fig. 2 im Schnitt,
Fig. 4 eine Zwischenbaugruppe einsprechend einer weiteren Ausführungsform der Erfindung in Explosivdarstellung, und
Fig. 5 die Zwischenbaugruppe nach Fig. 4 im zusammengebauten Zustand und im Schnitt.

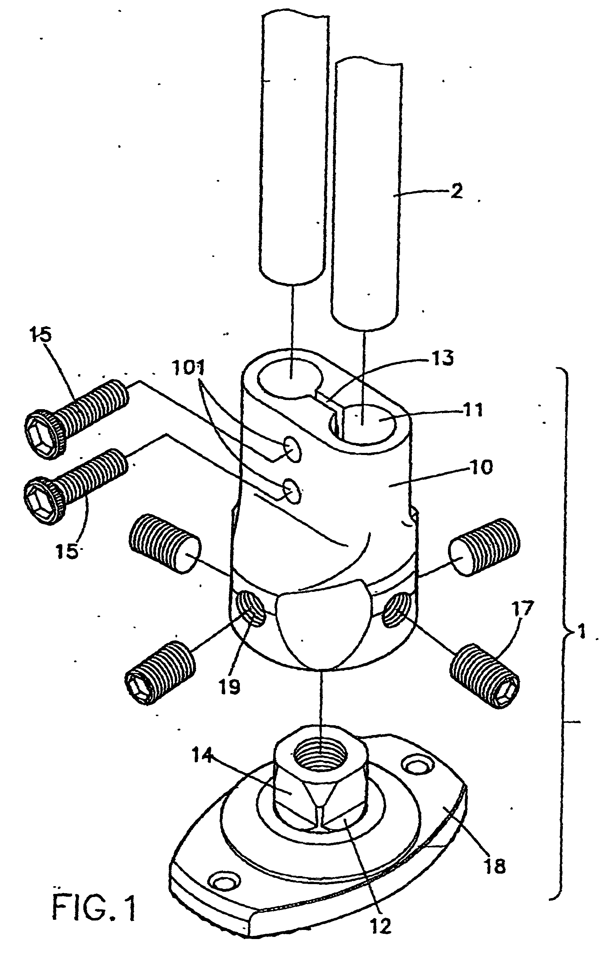

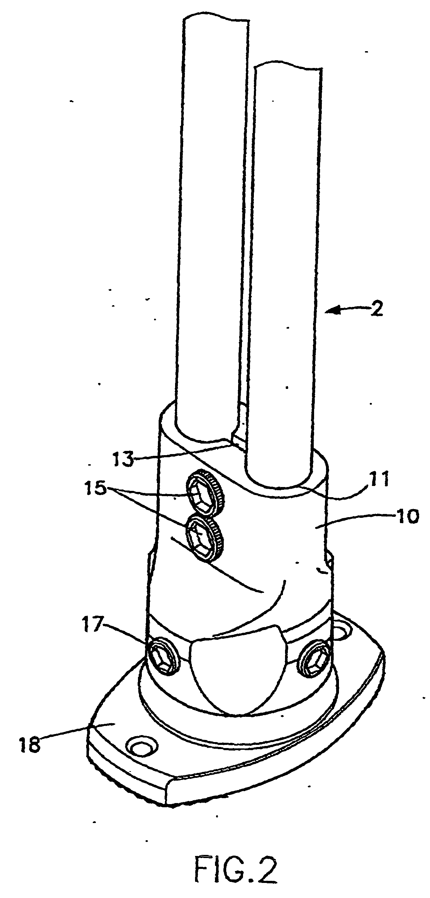

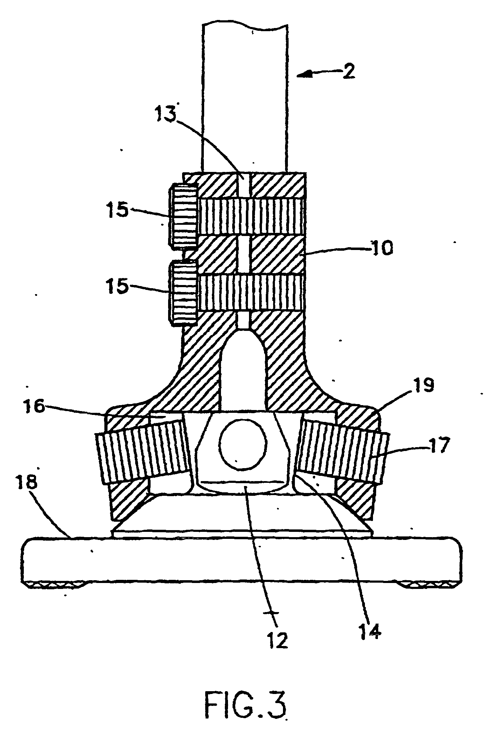

Wie aus Fig. 1, 2 und 3 ersichtlich ist, weist eine erfindungsgemäße Zwischenbaugruppe für eine Beinprothese als Hauptbestandteile ein Anschlussstück 1 und zwei Stäbe 2 auf.

Das Anschlussstück 1 weist einen ersten Anschlussteil 10 und eine zweiten Anschlussteil 18 auf. Die Stäbe 2 sind aus verstärktem Federmaterial hergestellt. Der erste Anschlussteil 10 weist zwei in axialer Richtung verlaufende und parallel zueinander angeordnete Aufnahmeöffnungen 11 auf, in denen jeweils die Stäbe 2 aufgenommen sind, einen axial verlaufenden und die beiden Aufnahmeöffnungen 11 verbindenden Spalt 13, zwei in vertikalem Abstand zueinander angeordnete und in Querrichtung durch den axial verlaufenden Spalt 13 gehende Befestigungsbohrungen 101, eine untere Kammer 16, sowie eine Mehrzahl von Gewindebohrungen 19, die leicht schräg und in radialer Richtung von der unteren Kammer 16 aus zum Außenrand verlaufen. Sind die Stäbe 2 in die jeweilige axial verlaufende Aufnahmeöffnung 11 eingesetzt, werden zwei Schraubenbolzen 15 in der jeweiligen Befestigungsbohrung 101 befestigt und fest angezogen, so dass sich der axial verlaufende schmale Spalt 13 schließt und die Stäbe 2 in dem ersten Anschlussteil 10 sicher festgelegt sind. Der zweite Anschlussteil 18 weist einen von seiner oberen Wand aus nach oben verlaufenden Polygon-Kopplungsschaft 12 auf. Der Polygon-Kopplungschaft 12 weist eine Mehrzahl abgeschrägter äußerer Seitenwände 14 auf, die jeweils schräg nach unten und innen verlaufen. Wenn der Polygon-Kopplungsschaft 12 in die untere Kammer 16 des ersten Anschlussteils 10 eingesetzt ist, werden ein Mehrzahl von Spannschrauben 17 in die Gewindebohrungen 19 geschraubt und gegen die abgeschrägten äußeren Seitenwände 14 arretiert, so dass der Polygon-Kopplungsschaft 12 des zweiten Anschlussteils 18 an dem ersten Anschlussteil 10 festgelegt ist.

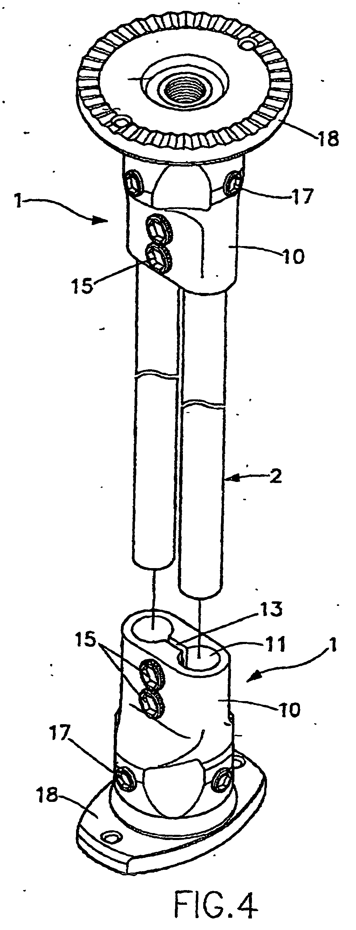

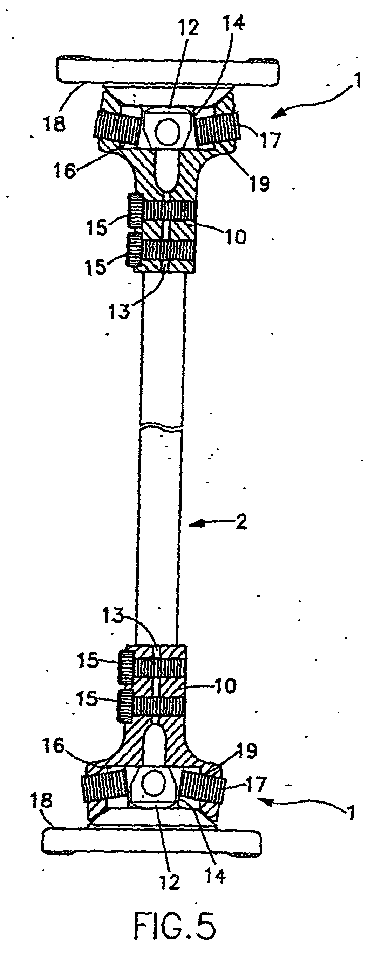

Wie aus Fig. 4 und 5 ersichtlich ist, weist die Zwischenbaugruppe einer weiteren Ausführungsform der Erfindung zwei Anschlussstücke 1 und zwei parallel zueinander verlaufende und zwischen den Anschlussstücken 1 befestigte Stäbe 2 auf. Die Anschlussstücke 1 und die Stäbe 2 dieser weiteren Ausführungsform ähneln dem Anschlussstück und den Stäben der aus Fig. 1,2 und 3 ersichtlichen Zwischenbaugruppe. Nach der Montage der Zwischenbaugruppe lässt sich jeweils eine Fußprothese und ein Knieprothese (nicht gezeigt) an den zweiten Anschlussteilen 18 der Anschlussstücke 1 befestigen.

Da die Einzelteile der Zwischenbaugruppe nicht mit Hilfe von Nieten aneinander festgelegt sind, ist die Zwischenbaugruppe abnehmbar und die Stäbe 2 sind problemlos entsprechend der jeweils gewünschten Länge austauschbar. Da die Stäbe 2 elastisch sind, werden sie beim Gehen abwechselnd gebogen und entspannt. Im gebogenen Zustand speichern die Stäbe 2 Energie, beim Loslassen setzen sie diese gespeicherte Energie jedoch sofort wieder frei.

Zusammenfassend schafft die Erfindung eine Zwischenbaugruppe mit zwei parallel zueinander angeordneten Stäben und einem an einem Ende an den Stäben befestigten Anschlussstück zum Verbinden der Stäbe mit einem Teil ener Beinprothese, wobei das Anschlussstück parallel zueinander angeordnete Aufnahmeöffnung aufweist, in denen die Stäbe jeweils aufgenommen sind, einen zwischen den Aufnahmeöffnungen ausgebildeten und diese miteinander verbindenden Spalt, eine Mehrzahl von entlang des Spalts in jeweils unterschiedlicher Höhe angeordneten Befestigungsbohrungen, sowie eine Mehrzahl von jeweils in den Befestigungsbohrungen befestigten Schraubenbolzen, die den Spalt schließen und die Stäbe in ihrer Lage festlegen.

## Patentansprüche

1. Beinprothese mit einer Zwischenbaugruppe mit mindestens zwei parallel zueinander angeordneten elastischen Stäben (2) und einem Anschlussstück (1), das an einem Ende der mindestens zwei Stäbe (2) befestigt ist, für das Verbinden der mindestens zwei Stäbe (2) mit einem Teil der Beinprothese, wobei das Anschlussstück (1) mindestens zwei Aufnahmeöffnungen (11) aufweist, die parallel zueinander angeordnet sind und in axialer Richtung zu einem seiner Enden verlaufen und in denen die mindestens zwei Stäbe entsprechend aufgenommen sind,
**dadurch gekennzeichnet**
**dass** das Anschlussstück (1) mindestens einen in axialer Richtung verlaufenden Spalt (13), der zwischen jeweils zwei benachbarten Aufnahmeöffnungen (11) ausgebildet ist und diese miteinander verbindet, eine Mehrzahl von Befestigungsbohrungen (101), die jeweils entlang des mindestens einen Spalts (13) angeordnet sind, sowie eine Mehrzahl von Schraubenbolzen (15), die jeweils in den Befestigungsbohrungen (101) befestigt sind und mit denen der mindestens eine Spalt (13) in seiner Spaltweite verringerbar ist und die mindestens zwei Stäbe (2) in ihrer Lage festlegbar sind.

## Claims

1. Leg prosthesis having an intermediate assembly with at least two mutually parallel elastic rods (2) and a connector piece (1) which is secured on one end of the at least two rods (2) for connecting the at least two rods (2) to a part of the leg prosthesis, the connector piece (1) having at least two receiving openings (11) which are arranged parallel to one another and extend in the axial direction to one of its ends and in which the at least two rods are correspondingly received, **characterized in that** the connector piece (1) has at least one gap (13) which extends in the axial direction and which is formed between two adjacent receiving openings (11) and connects these to one another, a plurality of securing bores (101) which are each arranged along the at least one gap (13), and a plurality of screw bolts (15) which are each secured in the securing bores (101) and with which the at least one gap (13) can be reduced in terms of its gap width and the at least two rods (2) can be fixed in their position.

## Revendications

1. Prothèse de jambe comprenant un module intermédiaire comportant au moins deux barres élastiques (2) parallèles et une pièce de raccordement (1) qui est fixée à une extrémité des deux barres (2) au moins afin de relier les deux barres (2) au moins à une pièce de la prothèse de jambe, la pièce de raccordement (1) comportant au moins deux ouvertures de réception (11) qui sont parallèles, qui s'étendent dans une direction axiale vers l'une de ses extrémités et qui reçoivent les au moins deux barres de façon appropriée,
**caractérisée en ce que**
la pièce de raccordement (1) comporte au moins une fente (13) qui s'étend dans une direction axiale et qui est toujours ménagée entre deux ouvertures de réception adjacentes (11) qu'elle relie, une pluralité de perçages de fixation (101) qui sont ménagés chacun le long d'au moins une fente (13), ainsi qu'une pluralité de boulons (15) qui sont fixés chacun dans un perçage de fixation (101) et qui permettent de verrouiller la largeur de la fente (13) au moins et de fixer la position des deux barres (2) au moins.
